# EUROPÄISCHE PATENTANMELDUNG

(11) **EP 1 482 025 A1**
(43) Veröffentlichungstag der Anmeldung: **01.12.2004**
(21) Anmeldenummer: 04012050.3
(22) Anmeldetag: 21.05.2004
(51) Int. Cl.: C11D 1/12, C11D 3/37, C11D 17/00

(54) **Fliessfähige Mischungen enthaltend Isethionat und Polyethylenglykol**

(30) Priorität: 27.05.2003 DE 10323963
(71) Anmelder: Clariant GmbH, 65929 Frankfurt am Main (DE)
(72) Erfinder: Henning, Torsten Dr, 65812 Bad Soden (DE); Simsch, Waltraud, 65779 Kelkheim (DE)
(74) Vertreter: Paczkowski, Marcus, Dr.

(57) **Zusammenfassung**

Die Erfindung betrifft fließfähige Mischungen enthaltend Isethionat und Polyethylenglykole. Die Mischungen können in flüssigen Produkten ohne weiteren Aufheiz- oder Solubilisierungsvorgang eingesetzt werden.

## Beschreibung

Die Erfindung betrifft bei Raumtemperatur fließfähige Mischungen enthaltend Isethionate. Isethionate sind als milde Tenside mit weiter Verbreitung in kosmetischen Mitteln und Waschmitteln bekannt.

Bisher stehen Isethionate auf dem Markt ausschließlich als feste Produkte zur Verfügung, die unter hohem Energie- und Zeitaufwand heiß unter Verwendung von Tensiden oder Lösungsmitteln gelöst werden müssen. Häufig tritt bei der Verarbeitung ein lästiges Stauben des Rohstoffs auf, was den Aufwand bei der Verwendung weiter erhöht, beispielsweise ist geeignete Schutzkleidung nötig oder es muss in speziell geschlossenen Anlagen gearbeitet werden.

Es bestand daher die Aufgabe, Isethionate in einer fießfähigen und pumpbaren Form zur Verfügung zu stellen.

Überraschend wurde nun gefunden, dass Mischungen aus Isethionaten und Polyethylenglykolen bei Raumtemperatur fließfähig und pumpbar sind.

Gegenstand der Erfindung sind fließfähige Mischungen enthaltend ein oder mehrere Isethionate der Formel I

RCOOCH₂CH₂SO₃M (I)

worin R C₆-C₃₀-, vorzugsweise C₈-C₂₂-Alkyl oder C₆-C₃₀-, vorzugsweise C₈-C₂₂-Alkenyl und M ein Alkalimetall-, Erdalkalimetall- oder Ammoniumion bedeutet sowie ein oder mehrere Polyethylenglykole der Formel II

H(OCH₂CH₂)ₙOH (II)

worin n eine Zahl von 4 bis 16, vorzugsweise 6 bis 16 und besonders bevorzugt 8 bis 12, bedeutet. Ganz außerordentlich bevorzugt ist n = 8.

Die erfindungsgemäßen Mischungen enthalten vorzugsweise 1 bis 90 Gew.-% Isethionat und 10 bis 99 Gew.-% Polyethylenglykol, besonders bevorzugt 10 bis 80 Gew.-% Isethionat und 20 bis 90 Gew.-% Polyethylenglykol. Ganz außerordentlich bevorzugt enthalten die erfindungsgemäßen Mischungen 25 bis 60 Gew.-% Isethionat und 25 bis 75 Gew.-% Polyethylenglykol.

In einer weiteren bevorzugten Ausführungsform enthalten die erfindungsgemäßen Mischungen auch ein oder mehrere Tenside. Die Tenside sind vorzugsweise ausgewählt aus Fettalkoholoxethylaten.

Wenn die erfindungsgemäßen Mischungen Tenside enthalten, sind diese vorzugsweise bis zu 70 Gew.-%, besonders bevorzugt von 10 bis 50 Gew.-% und außerordentlich bevorzugt von 15 bis 40 Gew.-% in den Mischungen enthalten.

In einer weiteren bevorzugten Ausführungsform enthalten die erfindungsgemäßen Mischungen auch Wasser. Wenn die erfindungsgemäßen Mischungen Wasser enthalten, ist dieses vorzugsweise bis zu 70 Gew.-%, besonders bevorzugt von 10 bis 50 Gew.-% und außerordentlich bevorzugt von 15 bis 40 Gew.-% in den Mischungen enthalten.

In einer weiteren bevorzugten Ausführungsform bestehen die erfindungsgemäßen Mischungen lediglich aus Isethionat und Polyethylenglykol sowie gegebenenfalls zusätzlich aus Substanzen ausgewählt aus Tensiden und Wasser.

Ein besonderer Vorteil der erfindungsgemäßen Mischungen, die wasserfrei sind oder zumindest wenig Wasser, d.h. bis zu ca. 40 Gew.-% Wasser, enthalten, ist, dass sie selbstkonservierend sind. Bei Verwendung dieser selbstkonservierenden Mischungen können die Kunden ihr eigenes Konservierungssystem für die Endprodukte verwenden und sind nicht auf das mitgelieferte Konservierungsmittel festgelegt.

Weiterhin unterstützt das Polyethylenglykol synergistisch die milde Reinigungswirkung des Isethionats indem es das Hautgefühl verbessert.

Die erfindungsgemäßen Mischungen zeigen zudem den Vorteil einer sehr guten Stabilität, d.h. es tritt keine Entmischung der erfindungsgemäßen Mischungen auf.

Die erfindungsgemäßen Mischungen können ohne einen weiteren Aufheiz- oder Solubilisierungsvorgang in flüssige Endprodukte wie beispielsweise Haarshampoos, Duschbäder oder Flüssigwaschmittel stabil eingearbeitet werden.

Die nachfolgenden Beispiele sollen den Gegenstand der Erfindung näher erläutern, ohne ihn darauf einzuschränken.

### Beispiele

### Beispiel 1

40 Gew.-% Hostapon® SCI-65 wurden mit 60 Gew.-% Polyglykol 200 USP gemischt. Die Mischung bleibt fließfähig.

### Beispiel 2

35 Gew.-% Hostapon® SCI-85 wurden mit 65 Gew.-% Polyglykol 300 gemischt. Die Mischung bleibt fließfähig.

### Beispiel 3

30 Gew.-% Hostapon® SCI-85 wurden mit 20 Gew.-% Hostapon® KCG und
50 Gew.-% Polyglykol 200 USP gemischt. Die Mischung bleibt fließfähig.

### Beispiel 4

45 Gew.-% Hostapon® SCI-85 wurden mit 27,5 Gew.-% Polyglykol 400 und
27,5 Gew.-% Genapol® L-3 bei Raumtemperatur gemischt. Die Mischung bleibt fließfähig.

### Anwendungsbeispiel A - Duschbad

Eine fließfähige Mischung aus 1,9 Gew.-% Hostapon® SCI-85 und 4,4 Gew.-% Polyglykol 200 USP wurde mit 78,1 Gew.-% Genapol® LRO flüssig und 15,6 Gew.-% Genagen® CAB in der Wärme gemischt und abgekühlt. Das resultierende Duschbad ist fließfähig und klar.

Verzeichnis der eingesetzten Produkte:

| | | |
|---|---|---|
| Polyglykol 200 USP | (Clariant GmbH) | PEG-4, Polyethylenglykol mit mittlerer Molmasse 200 g/mol |
| Polyglykol 300 | (Clariant GmbH) | PEG-6, Polyethylenglykol mit mittlerer Molmasse 300 g/mol |
| Polyglykol 400 | (Clariant GmbH) | PEG-8, Polyethylenglykol mit mittlerer Molmasse 400 g/mol |
| Hostapon® SCI-65 | (Clariant GmbH) | Sodium Cocoyl Isethionate |
| Hostapon® SCI-85 | (Clariant GmbH) | Sodium Cocoyl Isethionate |
| Hostapon® KCG | (Clariant GmbH) | Sodium Cocoyl Glutamate |
| Genapol® LRO | (Clariant GmbH) | Alkyldiglykolethersulfat, Na-Salz |
| Genapol® L-3 | (Clariant GmbH) | Laureth-3 |
| Genagen® CAB | (Clariant GmbH) | Cocamidopropyl Betaine |

## Patentansprüche

1. Mischung enthaltend ein oder mehrere Isethionate der Formel I
RCOOCH₂CH₂SO₃M (I)
worin R C₆-C₃₀-Alkyl oder C₆-C₃₀-Alkenyl und M ein Alkalimetall-, Erdalkalimetalloder Ammoniumion bedeutet sowie ein oder mehrere Polyethylenglykole der Formel II
H(OCH₂CH₂)ₙOH (II)
worin n eine Zahl von 4 bis 16 bedeutet.

2. Mischung nach Anspruch 1, **dadurch gekennzeichnet, dass** n 6 bis 16 bedeutet.

3. Mischung nach Anspruch 2, **dadurch gekennzeichnet, dass** n 8 bis 12 bedeutet.

4. Mischung nach einem oder mehreren der Ansprüche 1 bis 3, **dadurch gekennzeichnet, dass** sie 1 bis 90 Gew.-% Isethionat und 10 bis 99 Gew.-% Polyethylenglykol enthält.

5. Mischung nach Anspruch 4, **dadurch gekennzeichnet, dass** sie 10 bis 80 Gew.-% Isethionat und 20 bis 90 Gew.-% Polyethylenglykol enthält.

6. Mischung nach einem oder mehreren der Ansprüche 1 bis 5, **dadurch gekennzeichnet, dass** sie zusätzlich ein oder mehrere Tenside enthält.

7. Mischung nach einem oder mehreren der Ansprüche 1 bis 6, **dadurch gekennzeichnet, dass** sie zusätzlich Wasser enthält.

8. Verwendung einer Mischung nach einem oder mehreren der Ansprüche 1 bis 7 in Haarshampoos, Duschbädern oder Flüssigwaschmitteln.
